(19) Europäisches Patentamt
European Patent Office
Office européen des brevets

(11) Publication number: **0 407 594 A1**

(12) # EUROPEAN PATENT APPLICATION published in accordance with Art. 158(3) EPC

(21) Application number: **89911864.0**

(22) Date of filing: **26.10.89**

(86) International application number: **PCT/JP89/01099**

(87) International publication number: **WO 90/05134 (17.05.90 90/11)**

(51) Int. Cl.5: **C07D 239/06**, C07D 401/06, C07D 401/12, C07D 401/14, A01N 43/54

(30) Priority: **31.10.88 JP 275904/88**
**22.12.88 JP 324152/88**
**11.04.89 JP 189652/89**

(43) Date of publication of application: **16.01.91 Bulletin 91/03**

(84) Designated Contracting States: **AT BE CH DE FR GB IT LI LU NL SE**

(71) Applicant: **NIPPON SODA CO., LTD.**
**2-1, Ohtemachi 2-chome**
**Chiyoda-ku, Tokyo 100(JP)**

(72) Inventor: **KISHIMOTO, Takashi Odawara Research Center**
**Nippon Soda Co., Ltd. 345, Aza Yanagimachi**
**Takada, Odawara-shi Kanagawa 250-02(JP)**
Inventor: **ISHIMITSU, Keiichi Odawara Research Center**
**Nippon Soda Co., Ltd. 345, Aza Yanagimachi**
**Takada, Odawara-shi Kanagawa 250-02(JP)**
Inventor: **YAMADA, Tomio Odawara Research Center**
**Nippon Soda Co., Ltd. 345, Aza Yanagimachi**
**Takada, Odawara-shi Kanagawa 250-02(JP)**
Inventor: **TAKAKUSA, Nobuo Odawara Research Center**
**Nippon Soda Co., Ltd. 345, Aza Yanagimachi**
**Takada, Odawara-shi Kanagawa 250-02(JP)**
Inventor: **HATANO, Renpei Odawara Research Center**
**Nippon Soda Co., Ltd. 345, Aza Yanagimachi**
**Takada, Odawara-shi Kanagawa 250-02(JP)**

(74) Representative: **de Bruijn, Leendert C. et al**
**Nederlandsch Octrooibureau**
**Scheveningseweg 82 P.O. Box 29720**
**NL-2502 LS 's-Gravenhage(NL)**

(54) **NOVEL TETRAHYDROPYRIMIDINE DERIVATIVES, PROCESS FOR THEIR PREPARATION AND INSECTICIDES CONTAINING SAME AS ACTIVE INGREDIENTS.**

(57)

EP 0 407 594 A1

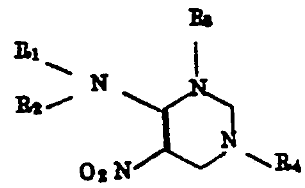

(I)

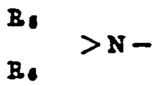 (α) $R_7O-$ (β) $R_8S(O)n-$ (ψ) 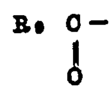 (δ)

$R_{10}COO-$ (ε)

The invention relates to compounds represented by general formula (I) (wherein $R_1$, $R_3$ and $R_4$, which may be the same or different, each represents an optionally substituted heterocyclic methyl group, hydrogen, an optionally substituted alkyl group, an optionally substituted alkenyl group, an optionally substituted alkynyl group, an optionally substituted phenyl group, **α, β, ψ, δ or ε** (wherein $R_5$, $R_6$, $R_7$, $R_8$, $R_9$ and $R_{10}$, which may be the same or different, each represents an optionally substituted heterocyclic methyl group, hydrogen, an optionally substituted alkyl group, an optionally substituted alkenyl group, an optionally substituted alkynyl group, or an optionally substituted phenyl group, and n represents 0, 1 or 2), $R_2$ represents an optionally substituted heterocyclic methyl group, hydrogen, an optionally substituted alkyl group, an optionally substituted alkenyl group, an optionally substituted alkynyl group, or an optionally substituted phenyl group, provided that the cases wherein $R_1$ represents hydrogen, $R_2$, $R_3$ and $R_4$ represent each a benzyl group and wherein $R_2$ represents hydrogen and $R_1$, $R_3$ and $R_4$ represent each a benzyl group are excluded), a process for preparing same, and insecticides containing same as active ingredients.

DESCRIPTION

Tetrahydropyrimidine derivative

Technical Field:

This invention relates to new tetrahydropyrimidine derivatives, their preparation methods and insecticides containing the said derivatives as active ingredients.

Background Art:

A large number of chemicals, for example, organic phosphorus insecticides such as parathion and malathion and carbamate insecticides such as carbaryl and methomyl, have been developed and put to practical use by research and development on insecticides over many years. These insecticides have played a very great role for the improvement of agricultural production. However, in recent years, some of these insecticides are regulated on their use because of problems such as environmental pollution due to residue or accumulation, or cause infestitation of resistant insect pests as a result of long-time use. Therefore, it is demanded to develop new chemicals which have excellent insecticidal characteristics over various types of insect pests including these resistant insect pests and which can be used safely.

As for compounds having a structure similar to that of the compounds of this invention, a compound represented by

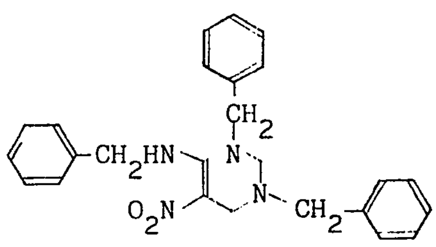

is described to be used as an intermediate for synthesis of heterocyclic compounds by M. Sone, et al in YAKUGAKU ZASSHI 97 (3) 262 - 267 (1977).

The object of this invention is to provide agricultural chemicals which can be advantageously synthesized industrailly, have certain effects and are

applicable safely.

Disclosure of the Invention:

This invention is a compound represented by general formula [I ]

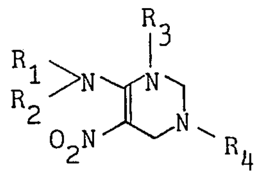

(I)

[where $R_1$, $R_3$ and $R_4$, same or different, are a heterocyclic methyl group which may be substituted, hydrogen, an alkyl group which may be substituted, an alkenyl group which may be substituted, an alkynyl group which may be substituted, a phenyl group which may be substituted, 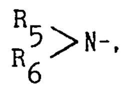, $R_7O-$, $R_8S(O)n-$, 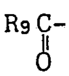, or $R_{10}COO-$ (where $R_5$, $R_6$, $R_7$, $R_8$, $R_9$ and $R_{10}$, same or different, are a heterocyclic methyl group which may be substituted, hydrogen, an alkyl group which may be substituted, an alkenyl group which may be substituted, an alkynyl group which may be substituted, or a phenyl group which may be substituted, and n is 0, 1 or 2), and $R_2$ is a heterocyclic methyl group which may be substituted, hydrogen, an alkyl group which may be substituted, an alkenyl group which may be substituted, an alkynyl group which may be substituted, or a phenyl group which may be substituted; but except the cases that $R_1$ is hydrogen and $R_2$, $R_3$ and $R_4$ are a benzyl group, and that $R_2$ is hydrogen and $R_1$, $R_3$ and $R_4$ are a benzyl group] , its preparation method and insecticide.

[Best Mode for Carrying of the Invention]

The compound of this invention is prepared as described below.

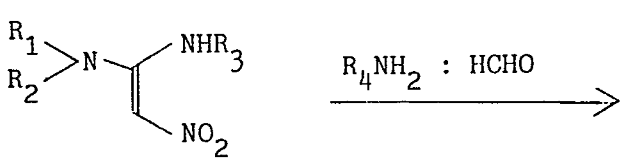


(I)

(II)

(where $R_1$, $R_2$, $R_3$ and $R_4$ are as defined above).

A compound represented by general formula 〔I〕 is obtained with good yield by the Mannich reaction in a way that a compound represented by general formula 〔II〕, $R_4NH_2$ 〔III〕 and HCHO are reacted in a solvent such as alcohols, at room temperature or by heating if necessary.

After the reaction is completed, a usual after-treatment gives the intended compound. The structure of the compound of this invention has been determined by such means as IR, NMR and MS.

This invention is further described in detail by reference to the following examples.

Example 1

1,3-Dimethyl-4-N-2-chloropyridi-5-ylmethyl-N-methylamino-5-nitro-1,2,3,6-tetrahydropyrimidine (Compound No. 24)

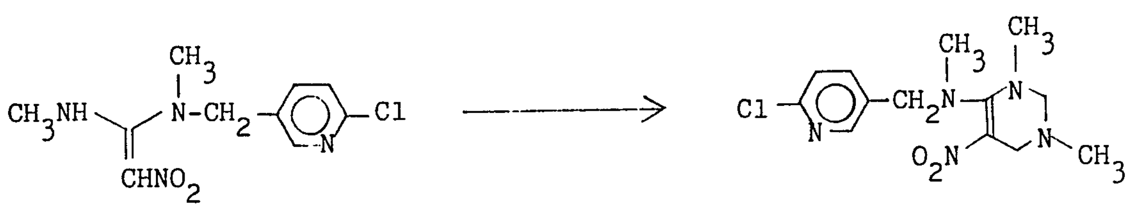

1.8g of 1-methylamino-1-N-methyl-N-2-chloropyridi-5-ylmethylamino-2-nitroethylene was dissolved in a solution containing 20mℓ of methanol and 4mℓ of water, into which 0.7g of 40% methylamine was dropped and further 1.2g of 37% HCHO was dropped, with stirring while cooling. After it, the solution was warmed up to room temperature and kept stirred for a night. After the reaction was completed, the solvents were distilled, and the obtained oily product was purified and isolated by column chromatography. 1.8g of the intended product was obtained. $n_D^{25.0}$ 1.6022

Example 2

1-Isopropyl-3-methyl-4-pyridi-3-ylmethylamino-5-nitro-1,2,3,6-tetrapyrimidine (Compound No. 16) and 1-isopropyl-3-pyridi-3-ylmethyl-4-methylamino-5-nitro-1,2,3,6-tetrahydropyrimidine (Compound No. 5)

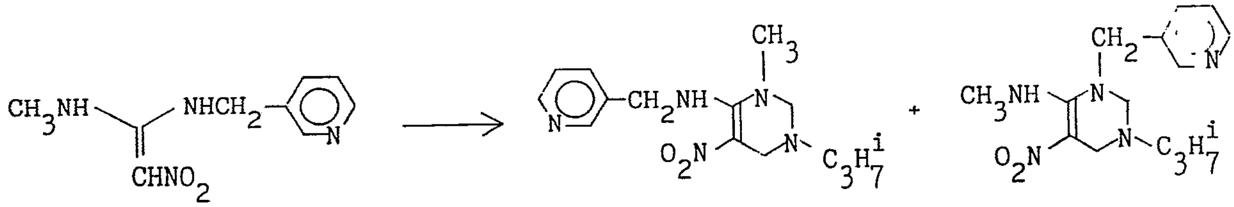

1.4g of 1-methyl-1-pyridi-3-ylmethylamino-2-nitroethylene was dissolved in a solution containing 10ml of methanol and 2ml of water, into which 0.5g of isopropylamine was dropped and further 1.2g of 37% HCHO was dropped, with stirring while cooling. After it, the solution was warmed up to room temperature and kept stirred for a night. After the reaction was completed, the solvents were distilled, and the obtained oily product was purified and isolated by column chromatography. 1.1g of the intended product (A) (m.p. 103 - 107 ℃) and 0.4g of (B) (m.p. 124 - 127 ℃) were obtained.

Typical examples of the compounds of this invention including those described above are shown in Table 1.

Table 1

| Compound No. | Structure Formula 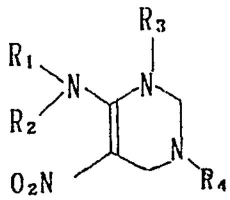 | | | | Physical Properties [ ] m.p. °C |
|---|---|---|---|---|---|
| | $R_1$ | $R_2$ | $R_3$ | $R_4$ | |
| 1 | H | $C_2H_5$ | $C_2H_5$ | $-CH_2-$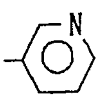 | [125 -128] |
| 2 | 〃 | 〃 | $-CH_2-$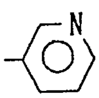 | 〃 | $n_D^{25.0}$ 1.6029 |
| 3 | 〃 | $-CH_2-$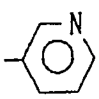 | $C_2H_5$ | 〃 | [ 95 - 98] |
| 4 | 〃 | 〃 | 〃 | $-CH_2-$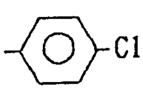$-Cl$ | $n_D^{25.0}$ 1.6038 |
| 5 | 〃 | $CH_3$ | $-CH_2-$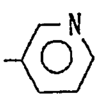 | $-C_3H_7^i$ | [124 -127] |
| 6 | 〃 | 〃 | 〃 | $-CH_2C{\equiv}CH$ | [141 -144] |
| 7 | 〃 | $C_2H_5$ | 〃 | $C_2H_5$ | [127 -130] |
| 8 | 〃 | 〃 | 〃 | $-CH_2C{\equiv}CH$ | $n_D^{25.0}$ 1.5994 |
| 9 | 〃 | 〃 | 〃 | $-CH_2-$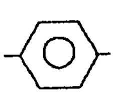$-Cl$ | $n_D^{25.0}$ 1.6050 |
| 10 | 〃 | $C_3H_7^i$ | 〃 | $C_2H_5$ | $n_D^{25.0}$ 1.5909 |
| 11 | $C_2H_5$ | $-CH_2-$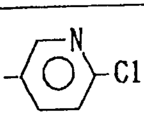$-Cl$ | $C_2H_5$ | $C_2H_5$ | $n_D^{25.0}$ 1.5967 |

| | | | | | |
|---|---|---|---|---|---|
| 12 | $C_2H_5$ | 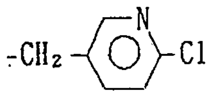 | $CH_3$ | $CH_3$ | $n_D^{25.0}$ 1.5948 |
| 13 | 〃 | 〃 | 〃 | $C_3H_7^i$ | $n_D^{25.0}$ 1.5797 |
| 14 | 〃 | 〃 | 〃 | 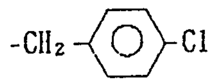 | $n_D^{25.0}$ 1.6061 |
| 15 | H | 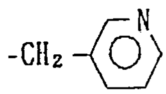 | 〃 | $CH_3$ | (126 -129) |
| 16 | 〃 | 〃 | 〃 | $C_3H_7^i$ | (103 -107) |
| 17 | 〃 | 〃 | 〃 | $-CH_2C\equiv CH$ | $n_D^{25.0}$ 1.6165 |
| 18 | 〃 | 〃 | $C_2H_5$ | $C_2H_5$ | (115 -118) |
| 19 | 〃 | 〃 | 〃 | $-CH_2C\equiv CH$ | ( 99 -102) |
| 20 | 〃 | 〃 | $-CH_2C\equiv CH$ | $C_2H_5$ | $n_D^{25.0}$ 1.5953 |
| 21 | 〃 | 〃 | 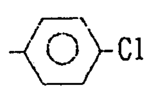 | 〃 | $n_D^{25.0}$ 1.6205 |
| 22 | 〃 | 〃 | 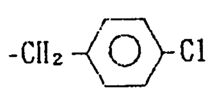 | 〃 | (149 -153) |
| 23 | 〃 | 〃 | $-N(CH_3)_2$ | 〃 | $n_D^{25.0}$ 1.5908 |
| 24 | $CH_3$ | 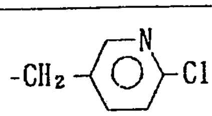 | $CH_3$ | $CH_3$ | $n_D^{25.0}$ 1.6022 |

| | | | | | |
|---|---|---|---|---|---|
| 25 | $CH_3$ | $-CH_2-$ 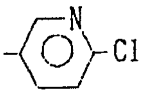 | $CH_3$ | $C_3H_7^i$ | $n_D^{25.0}$ 1.5952 |
| 26 | $C_3H_7^i$ | 〃 | 〃 | $CH_3$ | $n_D^{25.0}$ 1.5893 |
| 27 | H | 〃 | 〃 | 〃 | 〔141.5-145〕 |
| 28 | 〃 | 〃 | 〃 | $C_2H_5$ | 〔129 -131〕 |
| 29 | 〃 | 〃 | 〃 | $C_3H_7^i$ | 〔132 -134〕 |
| 30 | 〃 | 〃 | 〃 | $-CH_2CH=CH_2$ | 〔149 -151〕 |
| 31 | 〃 | 〃 | 〃 | $-CH_2C\equiv CH$ | 〔157 -160〕 |
| 32 | 〃 | 〃 | 〃 | $-CH_2-$ 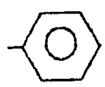 | 〔168-169.5〕 |
| 33 | 〃 | 〃 | $C_2H_5$ | $CH_3$ | 〔130 -133〕 |
| 34 | 〃 | 〃 | 〃 | $C_2H_5$ | |
| 35 | 〃 | 〃 | 〃 | $C_3H_7^i$ | |
| 36 | 〃 | 〃 | 〃 | $-CH_2CH=CH_2$ | |
| 37 | 〃 | 〃 | 〃 | $-CH_2C\equiv CH$ | |

| | | | | | |
|---|---|---|---|---|---|
| 38 | H | 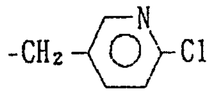 | $C_2H_5$ | 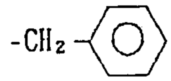 | |
| 39 | 〃 | 〃 | 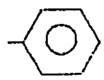 | $CH_3$ | |
| 40 | 〃 | 〃 | 〃 | $C_2H_5$ | |
| 41 | 〃 | 〃 | 〃 | $C_3H_7^i$ | |
| 42 | 〃 | 〃 | 〃 | $-CH_2CH=CH_2$ | |
| 43 | 〃 | 〃 | 〃 | $-CH_2C \equiv CH$ | |
| 44 | 〃 | 〃 | 〃 | 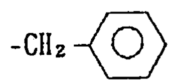 | |
| 45 | 〃 | 〃 | $C_3H_7^i$ | $C_2H_5$ | |
| 46 | 〃 | 〃 | 〃 | $-CH_2CH=CH_2$ | |
| 47 | 〃 | 〃 | 〃 | $-CH_2C \equiv CH$ | |
| 48 | 〃 | 〃 | 〃 | 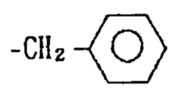 | |
| 49 | 〃 | 〃 | $-CH_2CH=CH_2$ | $C_2H_5$ | |
| 50 | 〃 | 〃 | 〃 | $-CH_2CH=CH_2$ | |

| | | | | | |
|---|---|---|---|---|---|
| 51 | H | -$CH_2$- 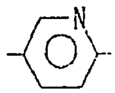 -Cl | -$CH_2CH=CH_2$ | -$CH_2C \equiv CH$ | |
| 52 | 〃 | 〃 | 〃 | -$CH_2$- 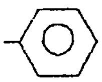 | |
| 53 | 〃 | 〃 | -$CH_2C \equiv CH$ | $C_2H_5$ | |
| 54 | 〃 | 〃 | 〃 | -$CH_2CH=CH_2$ | |
| 55 | 〃 | 〃 | 〃 | -$CH_2C \equiv CH$ | |
| 56 | 〃 | 〃 | 〃 | -$CH_2$- 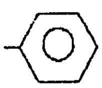 | |
| 57 | 〃 | 〃 | -$CH_2$- 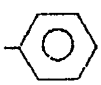 | $C_2H_5$ | |
| 58 | 〃 | 〃 | 〃 | -$CH_2CH=CH_2$ | |
| 59 | 〃 | 〃 | 〃 | -$CH_2C \equiv CH$ | |
| 60 | 〃 | 〃 | 〃 | -$CH_2$- 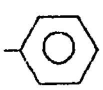 | |
| 61 | 〃 | 〃 | -$CH_2$- 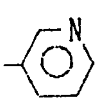 | $C_2H_5$ | |
| 62 | 〃 | 〃 | 〃 | -$CH_2CH=CH_2$ | |
| 63 | 〃 | 〃 | 〃 | -$CH_2C \equiv CH$ | |

| | | | | | |
|---|---|---|---|---|---|
| 64 | H | -$CH_2$ 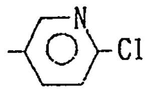 | -$CH_2$ 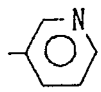 | -$CH_2$ 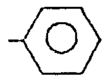 | |
| 65 | 〃 | 〃 | 〃 | 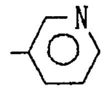 | |
| 66 | 〃 | 〃 | 〃 | 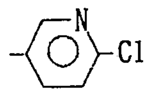 | |
| 67 | 〃 | 〃 | -$CH_2$ 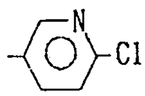 | $C_2H_5$ | |
| 68 | 〃 | 〃 | 〃 | $-CH_2CH{=}CH_2$ | |
| 69 | 〃 | 〃 | 〃 | $-CH_2C{\equiv}CH$ | |
| 70 | 〃 | 〃 | 〃 | -$CH_2$ 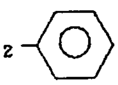 | |
| 71 | 〃 | 〃 | 〃 | 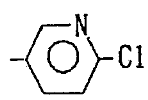 | |
| 72 | 〃 | 〃 | $CH_3$ | $CH_3$ | |
| 73 | 〃 | 〃 | 〃 | $C_2H_5$ | |
| 74 | 〃 | 〃 | $C_2H_5$ | $CH_3$ | |
| 75 | 〃 | 〃 | 〃 | $C_2H_5$ | |
| 76 | 〃 | 〃 | 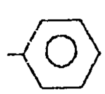 | $-CH_2C{\equiv}CH$ | |

| | | | | | |
|---|---|---|---|---|---|
| 77 | H | 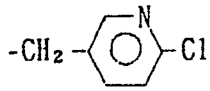 | 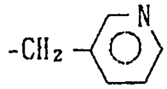 | $-CH_2CH{=}CH_2$ | |
| 78 | 〃 | 〃 | 〃 | 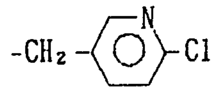 | |
| 79 | 〃 | 〃 | 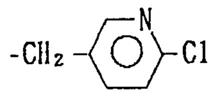 | 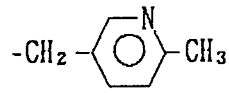 | |
| 80 | $CH_3$ | 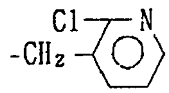 | $-CH_3$ | $-CH_3$ | |
| 81 | 〃 | 〃 | 〃 | $C_2H_5$ | |
| 82 | 〃 | 〃 | $C_2H_5$ | $CH_3$ | |
| 83 | 〃 | 〃 | 〃 | $C_2H_5$ | |
| 84 | 〃 | 〃 | 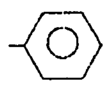 | $-CH_2C{\equiv}CH$ | |
| 85 | 〃 | 〃 | 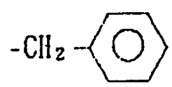 | $-CH_2CH{=}CH_2$ | |
| 86 | 〃 | 〃 | 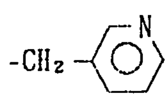 | 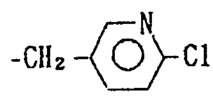 | |
| 87 | 〃 | 〃 | 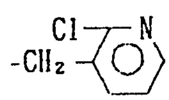 | 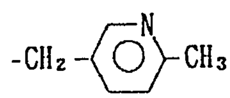 | |
| 88 | $C_2H_5$ | 〃 | $CH_3$ | $CH_3$ | |
| 89 | 〃 | 〃 | 〃 | $C_2H_5$ | |

| | | | | | |
|---|---|---|---|---|---|
| 90 | $C_2H_5$ | 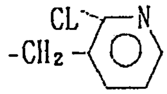 | $C_2H_5$ | $CH_3$ | |
| 91 | 〃 | 〃 | 〃 | $C_2H_5$ | |
| 92 | 〃 | 〃 | 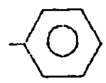 | $-CH_2C{\equiv}CH$ | |
| 93 | 〃 | 〃 | 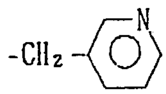 | $-CH_2CH{=}CH_2$ | |
| 94 | 〃 | 〃 | 〃 | 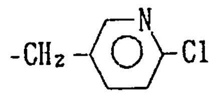 | |
| 95 | 〃 | 〃 | 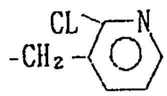 | 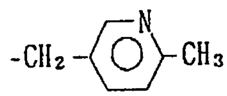 | |
| 96 | $-CH_2CH{=}CH_2$ | 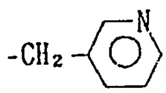 | $C_2H_5$ | 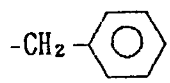 | |
| 97 | $-CH_2C{\equiv}CH$ | 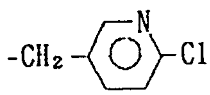 | $-CH_2CH{=}CH_2$ | 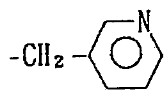 | |
| 98 | 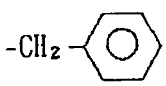 | $C_2H_5$ | 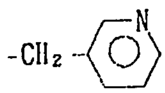 | $-CH_2C{\equiv}CH$ | |
| 99 | 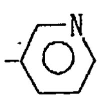 | 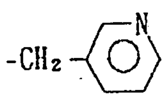 | $C_2H_5$ | $-CH_2CH{=}CH_2$ | |
| 100 | H | 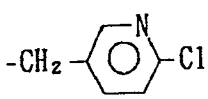 | $CH_3$ | 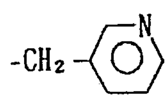 | |
| 101 | 〃 | 〃 | 〃 | 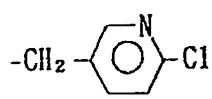 | (207 -209) |
| 102 | 〃 | 〃 | 〃 | 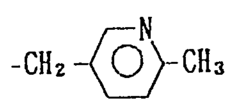 | |

| | | | | | |
|---|---|---|---|---|---|
| 103 | H | $CH_3$ | 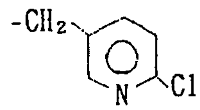 | H | |
| 104 | 〃 | 〃 | 〃 | $CH_3$ | (156 -159) |
| 105 | 〃 | 〃 | 〃 | $C_2H_5$ | (161 -163) |
| 106 | 〃 | 〃 | 〃 | $C_3H_7^i$ | (157 -158) |
| 107 | 〃 | 〃 | 〃 | 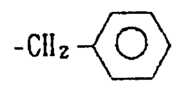 | (169-171.5) |
| 108 | 〃 | 〃 | 〃 | $-CH_2CH{=}CH_2$ | (167 -169) |
| 109 | 〃 | 〃 | 〃 | $-CH_2C{\equiv}CH$ | (187 -188) |
| 110 | 〃 | 〃 | 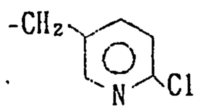 | H | |
| 111 | 〃 | 〃 | 〃 | $CH_3$ | |
| 112 | 〃 | 〃 | 〃 | $C_2H_5$ | |
| 113 | 〃 | 〃 | 〃 | 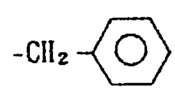 | |
| 114 | 〃 | 〃 | 〃 | $-CH_2CH{=}CH_2$ | |
| 115 | 〃 | 〃 | 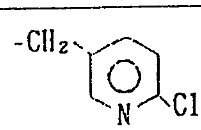 | 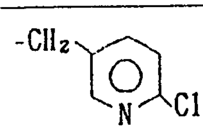 | (195 -197) |

| | | | | | |
|---|---|---|---|---|---|
| 116 | 〃 | 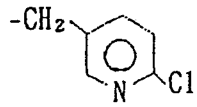 | H | $CH_3$ | |
| 117 | 〃 | 〃 | $CH_3$ | H | |
| 118 | 〃 | 〃 | H | H | |
| 119 | 〃 | 〃 | $-CH_2CH_2Cl$ | $CH_3$ | |
| 120 | 〃 | 〃 | $-CH_2CH_2OC_2H_5$ | 〃 | $n_D^{24.5}$ 1.5887 |
| 121 | 〃 | 〃 | $-CH_2CH_2N(CH_3)_2$ | 〃 | |
| 122 | H | H | 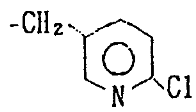 | H | |
| 123 | 〃 | 〃 | 〃 | $CH_3$ | |
| 124 | 〃 | 〃 | 〃 | $C_2H_5$ | |
| 125 | 〃 | 〃 | 〃 | $C_3H_7^i$ | |
| 126 | 〃 | 〃 | 〃 | 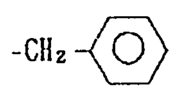 | |
| 127 | 〃 | 〃 | 〃 | $-CH_2CH{=}CH_2$ | |
| 128 | 〃 | 〃 | 〃 | $-CH_2C{\equiv}CH$ | |

| | | | | | |
|---|---|---|---|---|---|
| 129 | $CH_3$ | $CH_3$ | 〃 | H | |
| 130 | 〃 | 〃 | 〃 | $CH_3$ | |
| 131 | 〃 | 〃 | 〃 | $C_2H_5$ | |
| 132 | 〃 | 〃 | 〃 | $C_3H_7^i$ | |
| 133 | 〃 | 〃 | 〃 | 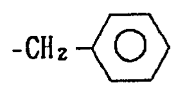 | |
| 134 | 〃 | 〃 | 〃 | $-CH_2CH=CH_2$ | |
| 135 | 〃 | 〃 | 〃 | $-CH_2C\equiv CH$ | |
| 136 | H | 〃 | 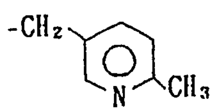 | $CH_3$ | |
| 137 | 〃 | 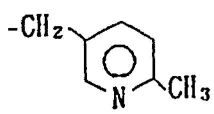 | $CH_3$ | 〃 | |
| 138 | 〃 | 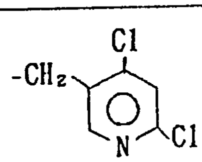 | 〃 | 〃 | |
| 139 | 〃 | 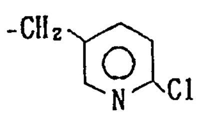 | $OC_2H_5$ | 〃 | (175 -177) |
| 140 | $-COCH_3$ | 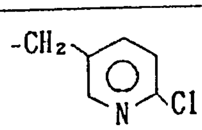 | $CH_3$ | $CH_3$ | $n_D^{24}$ 1.5570 |
| 141 | $-SCH_3$ | 〃 | 〃 | 〃 | |

| | | | | | |
|---|---|---|---|---|---|
| 142 | H | 〃 | 〃 | $-OC_2H_5$ | |
| 143 | 〃 | $-CH_2CH=CH_2$ | 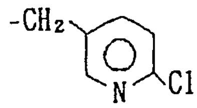 | $CH_3$ | |
| 144 | $CH_3$ | 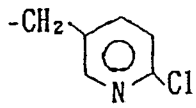 | H | 〃 | |
| 145 | 〃 | 〃 | $CH_3$ | $C_2H_5$ | |
| 146 | 〃 | 〃 | 〃 | 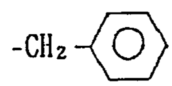 | |
| 147 | 〃 | 〃 | 〃 | $-CH_2CH=CH_2$ | |
| 148 | 〃 | 〃 | 〃 | $-CH_2C\equiv CH$ | |
| 149 | H | 〃 | $CH_3$ | H | |
| 150 | 〃 | 〃 | $C_2H_5$ | 〃 | |
| 151 | 〃 | 〃 | $-CH_2CH=CH_2$ | $CH_3$ | |
| 152 | 〃 | 〃 | 〃 | H | |
| 153 | 〃 | 〃 | $-CH_2C\equiv CH$ | 〃 | |
| 154 | 〃 | 〃 | 〃 | $CH_3$ | |

| | | | | | |
|---|---|---|---|---|---|
| 155 | 〃 | 〃 | 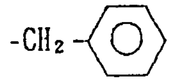 | H | |
| 156 | 〃 | 〃 | 〃 | $CH_3$ | |
| 157 | 〃 | 〃 | 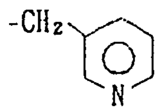 | H | |
| 158 | 〃 | 〃 | 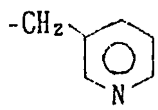 | $CH_3$ | |
| 159 | 〃 | 〃 | 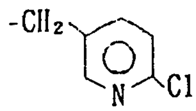 | 〃 | 〔149 -151〕 |
| 160 | $CH_3$ | 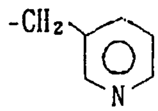 | $C_2H_5$ | $CH_3$ | 粘稠油 |
| 161 | H | 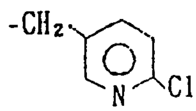 | $CH_3$ | $C_4H_9$ t | 〔184 -186〕 |
| 162 | 〃 | 〃 | 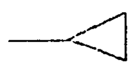 | $CH_3$ | 〔147 -149〕 |
| 163 | 〃 | 〃 | $C_2H_4OC_2H_5$ | $CH_3$ | $n_D^{24.5}$ 1.5887 |
| 164 | 〃 | 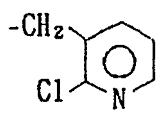 | $-C_2H_5$ | $CH_3$ | 〔153 -154〕 |
| 165 | 〃 | $CH_3$ | 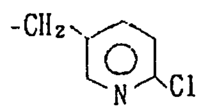 | $C_4H_9$ t | 〔163 -165〕 |
| 166 | 〃 | 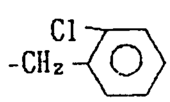 | $CH_3$ | $CH_3$ | 〔119 -121〕 |
| 167 | 〃 | 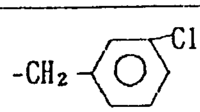 | 〃 | 〃 | 〔128 -130〕 |

| | | | | | |
|---|---|---|---|---|---|
| 168 | H | 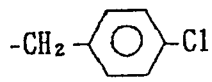 | $CH_3$ | $CH_3$ | 〔144 -146〕 |
| 169 | 〃 | 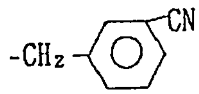 | 〃 | 〃 | 〔133 -134〕 |
| 170 | 〃 | 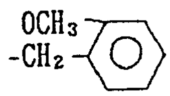 | 〃 | 〃 | 〔140 -142〕 |
| 171 | 〃 | 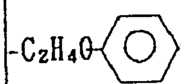 | 〃 | 〃 | $n_D^{25}$ 1.6128 |
| 172 | 〃 | 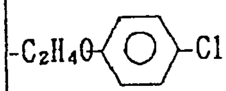 | 〃 | 〃 | 〔177 -179〕 |
| 173 | $COCH_3$ | 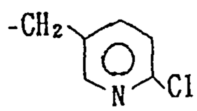 | 〃 | 〃 | |
| 174 | $SO_2CH_3$ | 〃 | 〃 | 〃 | |
| 175 | $CH_3$ | 〃 | $COCH_3$ | 〃 | |
| 176 | 〃 | 〃 | $SO_2CH_3$ | 〃 | |
| 177 | 〃 | 〃 | $CH_3$ | $COCH_3$ | |
| 178 | 〃 | 〃 | 〃 | $SO_2CH_3$ | |
| 179 | 〃 | 〃 | $COCH_3$ | $COCH_3$ | |
| 180 | 〃 | 〃 | $SO_2CH_3$ | $SO_2CH_3$ | |

| | | | | | |
|---|---|---|---|---|---|
| 181 | $CH_3$ | 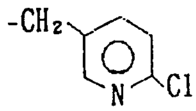 | $COCH_3$ | $SO_2CH_3$ | |
| 182 | 〃 | 〃 | $CH_3$ | $COOC_2H_5$ | |
| 183 | H | 〃 | $COCH_3$ | $CH_3$ | |
| 184 | 〃 | 〃 | $SO_2CH_3$ | 〃 | |
| 185 | 〃 | 〃 | $CH_3$ | $COCH_3$ | |
| 186 | 〃 | 〃 | 〃 | $SO_2CH_3$ | |
| 187 | $COCH_3$ | 〃 | $COCH_3$ | $COCH_3$ | |
| 188 | $COOC_2H_5$ | 〃 | $CH_3$ | $CH_3$ | |

The compounds of this invention exhibit high insecticidal activities against various species of insect pests such as cutworms, diamondback moth, aphids, leafhoppers and planthoppers. In recent years the decrease of the control effects of organophosphorus and carbamate insecticides, which is caused by the development of resistance to these insecticides, has become serious problem. In such situations, the development of new insecticides which is effective on the resistant pests has been desired. The compounds of this invention possess superior insecticidal activities against not only susceptible strains but also resistant ones.

The insecticides covered by this invention contain as active ingredients one or more types of the compounds as expressed by the general formula (1). These active ingredients, which the compounds are, may be used as-produced but normally they are used in any of the forms which ordinary agricultural chemicals can take, namely wettable powder, dust, emulsifiable concentrate, suspension concentrates or other formulations. For additives and carriers are used soybean flour, wheat flour or other vegetable flours, diatomaceous earth, apatite, gypsum, talc, pyrophyllite, clay or other fine mineral powders, when solid formulations are intended.

When liquid formulations are intended, then for solvents are used kerosene, mineral oil, petroleum, solvent naphtha, xylene, cyclohexane, cyclohexanone, dimethylformamide, dimethylsulfoxide, alcohol, acetone, water, etc. A surface active agent may, if necessary, be added in order to give a homogeneous and suitable formulation. The wettable powders, emulsifiable concentrates, suspension concentrates, etc. thus obtained are diluted with water into suspensions or emulsions of a prescribed concentration, before they are actually sprayed on plants in the field. In the case of dusts or granules, they are directly applied without further processing.

It goes without saying that the compound(s) of this invention is effective even alone, but it can be used by mixing with various types of insecticides, acaricides and fungicides.

Typical examples of acaricides and insecticides which can be used by mixing with the compounds of this invention are described below:

Acaricides (fungicides):

chlorobenzilate, chloropropylate, proclonol, bromopropylate, dicofol, dinobuton, binapacryl, chlordimeform, amitraz, propargite, PPPS, benzoximate, hexythiazox, fenbutatin oxide, polynactins, chinomethionat, thioquinox, chlorfenson, tetradifon, phenproxide, avermectins, calcium polysulfide, clofentezine, flubenzimine, thiophanate-methyl, benomyl, thiram, iprobenfos, edifenfos, fthalide, probenazole, isoprothiolane, chlorothalonil, captan, polyoxin-B, blasticidin-S, kasugamycin, validamycin, tricyclazole, pyroquilon, phenazine oxide, mepronil, flutolanil, pencycuron, iprodione, hymexazole, metalaxyl, triflumizole, diclomezine, tecloftalam.

Organophosphorus-type and carbamate-type insecticides (acaricides):

fenthion, fenitrothion, diazinon, chlorpyrifos, oxydeprofos, vamidothion, phenthoate, dimethoate, formothion, malathion, trichlorfon, thiometon, phosmet, menazon, dichlorvos, acephate, EPBP, dialifos, parathion-methyl, oxydemethon-methyl, ethion, aldicarb, propoxur, methomyl, fenobucarb, BPMC.

Pyrethroid-type insecticides (acaricides):

permethrin, cypermethrin, deltamethrin, fenvalerate, fenpropathrin, pyrethrins, allethrin, tetramethrin, resmethrin, parthrin, dimethrin, proparthrin, bifenthrin, prothrin, fluvalinate, cyfluthrin, cyhalothrin, flucythrinate, ethofenprox, cycloprothrin, tralomethrin.

Benzoylphenylurea-type and other types insencticides:

diflubenzuron, chlorfluazuron, triflumuron, teflubenzuron, buprofezin. Machine oil.

Same examples of the formulations are given below. The carriers, surface-active agents, etc. that are added, however, are not limited to these Examples.

Example 3 : Emulsifiable concentrate

| | |
|---|---|
| The compound of this invention | 10 parts |
| Alkylphenyl polyoxyethylene | 5 parts |
| Dimethyl formamide | 50 parts |
| Xylene | 35 parts |

These components are mixed and dissolved and, for use in spraying, the liquid mixture is water-diluted into an emulsion.

Example 4 : Wettable powder

| | |
|---|---|
| The compound of this invention | 20 parts |
| Higher alcohol sulfuric ester | 5 parts |
| Diatomaceous earth | 70 parts |
| Silica | 5 parts |

These components are mixed and ground to fine powders, which for use in spraying, are water-diluted into a suspension.

Example 5 : Dust

| | |
|---|---|
| The compound of this invention | 5 parts |
| Talc | 94.7 parts |
| Silica | 0.3 parts |

These are mixed and ground and used as-ground in spraying.

Example 6 : Granule

| | |
|---|---|
| The compound of this invention | 5 parts |
| Clay | 73 parts |
| Bentonite | 20 parts |
| Sodium dioctylsulfosuccinate | 1 part |
| Sodium phosphate | 1 part |

The above compounds are granulated, and applied as it is when used.

Industrial applicability:

The tests below show the insecticidal activity of the compounds of this invention.

Test 1 Efficacy for cotton aphid

30 to 50 insects of cotton aphid per plot were inoculated using a small brush on cucumber leaves which were seeded in pots, 10cm in diameter, and 10 days old after germination. A day later, wounded insect pests were removed, and a chemical solution, which was prepared in the way that the emulsifiable concentrate described in Example 3 of the above example of insecticide was diluted with water to 125 ppm of compound concentration according to the prescription, was sprayed. The pots were placed in a thermostatic room at temperature of 25°C and humidity of 65%. The number of survival pests was counted after 7 days and the control efficacy was calculated by comparing with that of untreated plot. The results are shown in Table 2.

Table 2

| Compound No. | Control efficacy after 7 days<br>125 ppm |
|---|---|
| 2 | 100 % |
| 3 | 100 |
| 5 | 100 |
| 6 | 100 |
| 7 | 100 |
| 8 | 100 |
| 9 | 100 |
| 12 | 100 |
| 13 | 100 |
| 14 | 100 |
| 15 | 100 |
| 16 | 100 |
| 18 | 100 |
| 19 | 100 |
| 20 | 100 |
| 23 | 100 |
| 24 | 100 |
| 27 | 100 |
| 28 | 100 |
| 29 | 100 |
| 30 | 100 |
| 31 | 100 |
| 32 | 100 |
| 33 | 100 |
| 101 | 100 |
| 104 | 100 |
| 105 | 100 |
| 106 | 100 |
| 107 | 100 |
| 108 | 100 |
| 109 | 100 |
| 115 | 100 |
| 138 | 100 |
| 159 | 100 |

| | |
|---|---|
| 161<br>162<br>165<br>168<br>171 | 100<br>100<br>100<br>100<br>100 |
| Comparative Compound A<br>〃 B | 0<br>0 |

Comparative Compound A :

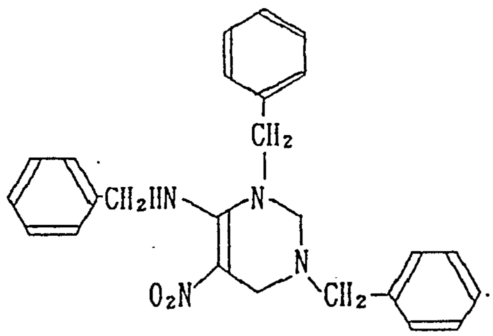

Comparative Compound B :

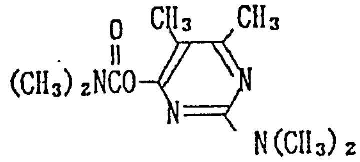

(pirimicarb)

Test 2 Efficacy for green rice leafhopper

Rice seedlings of 7 days old after germination were immersed in a chemical solution, which was prepared in the way that the emulsifiable concentrate described in Example 3 of the above example of insecticide was diluted with water to 125 ppm of compound concentration according to the prescription, for 30 seconds. After dried in air, the treated seedlings were placed in test tubes and 10 insects of 3rd-instar larvae of green rice leafhopper resistant to the organophosphorus and carbamate insecticides were inoculated. The tubes were covered with gauze, and placed in a thermostatic room at temperature of 25°C and humidity of 65%. The mortality was checked after 5 days.

The results are shown in Table 3.

Table 3

| Compound No. | % mortality after 5 days |
|---|---|
| | 125 ppm |
| 1 | 100 % |
| 5 | 100 |
| 6 | 100 |
| 12 | 100 |
| 13 | 100 |
| 14 | 100 |
| 15 | 100 |
| 17 | 100 |
| 24 | 100 |
| 25 | 100 |
| 26 | 100 |
| 27 | 100 |
| 28 | 100 |
| 29 | 100 |
| 30 | 100 |
| 31 | 100 |
| 32 | 100 |
| 33 | 100 |
| 101 | 100 |
| 104 | 100 |
| 105 | 100 |
| 106 | 100 |
| 107 | 100 |
| 108 | 100 |
| 109 | 100 |
| 115 | 100 |
| 138 | 100 |
| 159 | 100 |
| 161 | 100 |
| 162 | 100 |

| | |
|---|---|
| 165<br>166<br>167<br>168<br>171 | 100<br>100<br>100<br>100<br>100 |
| Comparative Compound A<br>〃 C | 0<br>0 |

Comparative Compound : The same as Test 1

Comparative Compound C :

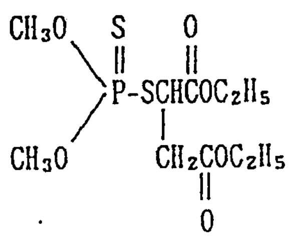

(malathion)

Test 3 Efficacy for rice armyworm

The test compounds were formulated into the wettable powder in the same manner as

Example 4. The compounds were diluted with water to 125ppm. A maize leaf was immersed in the chemical solution for 30 seconds. After air-dried, the treated leaf was placed in a petri dish and five 3rd- instar larvae of rice armyworm were inoculated. The petri dishes were covered with glass lids, and placed in a thermostatic room at 25°C and 65% relative humidity. The mortality was checked 5 days after treatment. Two replications were conducted in the each test. The results are shown in Table 4.

Table 4

| Compound No. | % mortality after 5 days |
| --- | --- |
| | 125 ppm |
| 5 | 100 % |
| 6 | 100 |
| 15 | 100 |
| 16 | 100 |
| 27 | 100 |
| 28 | 100 |
| 29 | 100 |
| 30 | 100 |
| 31 | 100 |
| 32 | 100 |
| 104 | 100 |
| 105 | 100 |
| 106 | 100 |
| 107 | 100 |
| 108 | 100 |
| 109 | 100 |
| 115 | 100 |
| 161 | 100 |
| 165 | 100 |
| Comparative Compound A | 20 |
| Comparative Compound D | 40 |

Comparative Compound A: The same as Test 1

Comparative Compound D:

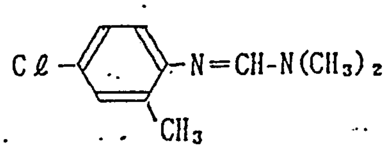

(chlordimeform)

## Claims

(1) A compound represented by general formula 〔I〕

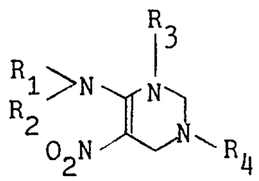

(I)

〔where $R_1$, $R_3$ and $R_4$, same or different, are a heterocyclic methyl group which may be substituted, hydrogen, an alkyl group which may be substituted, an alkenyl group which may be substituted, an alkynyl group which may be substituted, a phenyl group which may be substituted, $\genfrac{}{}{0pt}{}{R_5}{R_6}\!>\!N-$, $R_7O-$, $R_8S(O)n-$, $R_9\underset{\underset{O}{\|}}{C}-$,or $R_{10}COO-$ (where $R_5$, $R_6$, $R_7$, $R_8$, $R_9$ and $R_{10}$ , same or different, are a heterocyclic methyl group which may be substituted, hydrogen, an alkyl group which may be substituted, an alkenyl group which may be substituted, an alkynyl group which may be substituted, or a phenyl group which may be substituted, and n is 0, 1 or 2), and $R_2$ is a heterocyclic methyl group which may be substituted, hydrogen, an alkyl group which may be substituted, an alkenyl group which may be substituted, an alkynyl group which may be substituted, or a phenyl group which may be substituted; but except the cases that $R_1$ is hydrogen and $R_2$, $R_3$ and $R_4$ are a benzyl group, and that $R_2$ is hydrogen and $R_1$,$R_3$ and $R_4$ are a benzyl group〕.

(2) A process for the preparation of compound represented by general formula 〔I〕

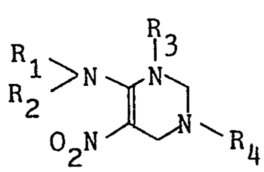

(I)

(where $R_1$, $R_2$, $R_3$ and $R_4$ are as defined above), which comprises reacting a compound represented by general formula 〔II〕

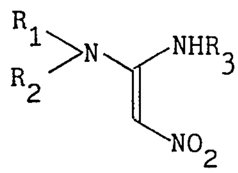

(where $R_1$, $R_2$ and $R_3$ are as defined above), with a compound represented by general formula 〔III〕 $_4NH_2$ (where $R_4$ is as defined above) and HCHO.

(3) An insecticide which comprises containing one or two or more compounds represented by general formula 〔I〕

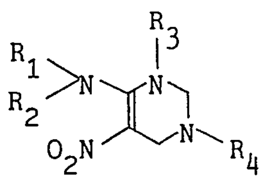

(I)

(where $R_1$, $R_2$, $R_3$ and $R_4$ are as defined above), as effective components.

# INTERNATIONAL SEARCH REPORT

International Application No PCT/JP89/01099

**I. CLASSIFICATION OF SUBJECT MATTER** (if several classification symbols apply, indicate all) [6]

According to International Patent Classification (IPC) or to both National Classification and IPC

Int. $Cl^5$ C07D239/06, 401/06, 401/12, 401/14, A01N43/54

**II. FIELDS SEARCHED**

Minimum Documentation Searched [7]

| Classification System | Classification Symbols |
|---|---|
| IPC | C07D239/06, 401/06, 401/12, 401/14, A01N43/54 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | Yakugaku Zasshi (YAKUGAKU ZASSHI), Vol.97, No.3, March 1977 (Tokyo) Sone Masakatsu and three others [1-Nitro-2,2-bis(methylthio) ethylene no Hanno] P.262 - 267 | 1, 2, 3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 19, 1990 (19. 01. 90) | February 5, 1990 (05. 02. 90) |
| **International Searching Authority** | **Signature of Authorized Officer** |
| Japanese Patent Office | |